# EUROPEAN PATENT SPECIFICATION

(11) **EP 2 645 103 B1**
(45) Date of publication and mention of the grant of the patent: **23.03.2016**
(21) Application number: 11843001.6
(22) Date of filing: 22.11.2011
(51) Int. Cl.: G01N 33/68, G01N 33/543

(54) **GLYCOPEPTIDE ARRAY**
GLYCOPEPTIDARRAY
RÉSEAU DE GLYCOPEPTIDES

(30) Priority: 26.11.2010 JP 2010264099
(43) Date of publication of application: 02.10.2013
(73) Proprietor: National University Corporation Hokkaido University, Sapporo-shi, Hokkaido 060-0808 (JP); Sumitomo Bakelite Company Limited, Shinagawa-ku Tokyo 140-0002 (JP)
(72) Inventor: NISHIMURA, Shin-Ichiro, Hokkaido 060-0808 (JP); MATSUSHITA, Takahiko, Hokkaido 060-0808 (JP); SHIMAOKA, Hideyuki, Tokyo 140-0002 (JP); TAKADA, Wataru, Tokyo 140-0002 (JP); IGARASHI, Kohta, Tokyo 140-0002 (JP); ABE, Midori, Tokyo 140-0002 (JP); ABE, Hiroki, Tokyo 140-0002 (JP); FUKUSHIMA, Masao, Tokyo 140-0002 (JP)
(74) Representative: Schnappauf, Georg
(86) International application number: PCT/JP2011/076889
(87) International publication number: WO 2012/070564

(56) References cited:
- JP-A- 2007 326 920
- JP-A- 2007 326 920
- JP-A- 2007 527 539
- JP-A- 2011 191 286
- US-A1- 2007 269 895
- BEDAIR MOHAMED ET AL: "Lectin affinity chromatography using porous polymer monolith assisted nanoelectrospray MS/MS.", THE ANALYST DEC 2006, vol. 131, no. 12, December 2006 (2006-12), pages 1316-1321, XP008168225, ISSN: 0003-2654
- TAKAHIKO MATSUSHITA ET AL.: 'Shikkan Tokuiteki Epitope Tansaku o Shiko shita MUC1 To Peptide Microarray no Kaihatsu' DAI 30 KAI THE JAPANESE SOCIETY OF CARBOHYDRATE RESEARCH NENKAI YOSHISHU vol. SHIKKAN, 27 June 2011, page 75, XP008168150

## Description

### Technical Field

The present invention relates to a glycopeptide array capable of detecting a binding between a substance to be detected and a glycopeptide.

Priority is claimed on Japanese Patent Application No. 2010-264099, filed November 26, 2010.

### Background Art

Traditionally, attempts to evaluate gene activity and to decode a physiological process at a molecular level of a drug effect have focused on genomics. However, proteomics provides more detailed information on the biological functions of a cell. Proteomics includes qualitative and quantitative measurement of gene activity by detecting and quantitating gene expression at a protein level, rather than a gene level. In addition, research of matters which are not encoded in a genome, such as post-translational modification of proteins and interaction between proteins, is included. On the other hand, in recent years, a sugar chain has attracted attention as the third chain in vivo. Particularly, the relationship with cell differentiation and canceration, immune reaction and fertilization, and the like has been researched and an attempt to make new medical drugs and medical materials has continued. Moreover, the sugar chain is a receptor for a number of toxins, viruses, bacteria and the like and has attracted attention as a cancer marker. Lately, an interaction of the sugar chain with amyloid protein, which is considered a factor in cancer metastasis and Alzheimer's disease, has been reported.

A genome structure that is the "blueprint of life" has been clarified and a vast amount of genome information is available nowadays. Proteomics and sugar chain research has become more popular, and accordingly, there has been a demand for speed and high efficiency (high throughput) in physiologically active substance detection. Therefore, the research and development of a substrate on which a physiologically active substance having sugars such as an antibody, a glycopeptide, or a sugar chain is immobilized as a target molecular array has been advancing. A protein chip including an antibody chip, a sugar chain chip and the like is a good example of the substrate.

However, since the protein chip and the like have been generally developed as an extension of a DNA chip, studies have been conducted on immobilization of the protein on a glass substrate or the molecule for capturing the protein on a chip surface (for example, refer to PTL 1).

There is a sandwich method which is generally used in detection and quantitative determination of a specimen such as a protein. This method is a method in which an antibody (primary antibody) is bound to a solid phase, a target protein is trapped by an insolubilized antibody, and then, a labeled antibody (secondary antibody) which recognizes and binds to another epitope of the target protein is allowed to react to measure the labeled substance so that the protein is quantitatively measured (for example, refer to PTL 1). However, in this method, for example, when detection of many kinds of proteins is attempted at one time as a protein chip, plural kinds of antibodies not competing with each other are required for each target protein, and thus there are many problems to be solved for optimization of conditions.

In addition, the molecule that captures the protein (hereinafter, referred to as "capture molecule") is immobilized on the substrate, and then, for example, as in the sandwich method, the molecule reacts with another protein (in the case of an antigen antibody reaction, corresponding to an antigen) on the surface, and further, reacts with the labeled protein to finally detect the protein by a detector and the like, in a portion in which the capture molecule is not immobilized, when a protein other than the molecule, that is, the antigen or the labeled protein is immobilized, the immobilization of the antigen or the target protein becomes noise at the time of detection and is a cause of decreasing the signal-to-noise ratio (S/N ratio) so that detection accuracy is lowered (for example, refer to PTL 2). Particularly, in the test which is conducted by the present inventors, there is a tendency of increasing non-specific adsorption of contaminating protein and noise and decreasing the S/N ratio in blood serum and blood plasma used in a clinical test and the like.

Accordingly, in the normal sandwich method, the coating of an adsorption-inhibiting agent is performed to prevent the non-specific adsorption of the antigen or the secondary antibody after the primary antibody is immobilized. However, the ability of preventing the non-specific adsorption of the antigen or the secondary antibody is not sufficient. In addition, since the coating of an adsorption-inhibiting agent is performed after the primary antibody is immobilized, there is a problem in that the immobilized proteins are coated with the adsorption-inhibiting agent, thereby, the immobilized proteins cannot react with the second antibody. Therefore, there has been a demand for a biochip with no adsorption-inhibiting agent coating after immobilizing the primary antibody and less amount of non-specific adsorption of the physiologically active substance.

On the other hand, when the primary antibody is immobilized, it is important that the primary antibody be immobilized so that the antigen-binding portion of the antibody easily functions. For example, PTL 2 discloses a method for binding a specified immobilized substance to a fine hole formed on a substrate. However, in this method, when the immobilized substance is an antibody, the substrate and peptide are bound by using an amino group terminal (N terminal), and hence, the antigen-binding portion does not sufficiently function in some cases.

Here, the present applicant developed a polymer compound for bioassay capable of inhibiting non-specific adsorption or binding of a biological molecule and immobilizing various physiologically active substances without the coating of an adsorption-inhibiting agent, and a substrate using the same (PTL 3). The surface of the substrate is coated with the polymer compound containing three units of a unit having a phosphorylcholine group to inhibit the non-specific adsorption of a biological molecule, a unit having a hydrophobic group to be bound to the substrate, and a unit having a primary amino group to bind a target substance, and the target substance can be immobilized on the substrate by the binding between the primary amino group of the polymer compound and an aldehyde group of the target substance.

However, when a glycopeptide is immobilized on the substrate, an improvement is desired to increase efficiency of immobilization while avoiding the mixing of a side reaction product and a contaminated substance.

In addition, NPL 1 discloses an array with a glycopeptide immobilized on a glass substrate by a chemical reaction in which an amino group contained in the glycopeptide reacts with the surface of the glass substrate which is activated using N-hydroxysuccinimide (NHS) ester to form an amide binding. However, in the case where the glycopeptide array is produced by this method, since plural reaction points against the substrate are present in the glycopeptide, the orientation control of the immobilized glycopeptide is difficult, and there are problems of mixing of a side reaction product and a contaminated substance and non-specific adsorption of a substance to be detected to the substrate.

NPL 2 describes that an affinity porous polymer monolith is utilized as a nanoelectrospray emitter as well as an online affinity capture column for the preconcentration of glycans.

PTL 4 discloses a method for identifying and quantifying polyglycopeptides in a sample.

### Citation List

### Patent Literature

[PTL 1 Japanese Unexamined Patent Application, First Publication No. 2001-116750
[PTL 2] Japanese Unexamined Patent Application, First Publication No. 2005-214889
[PTL 3] Japanese Unexamined Patent Application, First Publication No. 2007-326920
[PTL 4] US Patent Application No. 2007/0269895 A1

### Non-Patent Literature

[NPL 1] Hans H. et al., Cancer Res; 70(4) February 15, 2010
[NPL 2] Mohamed Bedair et al., Analyst; 131(12) December 2006

### Summary of the Invention

### Technical Problem

The present invention is made in consideration of the above-described circumstances and an object thereof is to provide an array which has a glycopeptide immobilized and which is useful for detection of binding between a substance to be detected and a glycopeptide. Another object of the present invention is to provide an array which can inhibit non-specific adsorption or binding of a substance to be detected without coating of any adsorption-inhibiting agent and which has the glycopeptide immobilized thereon.

### Solution to the Problem

As a result of extensive research to achieve the objects, the present inventors have found that a glycopeptide, which is bound to a molecule having a carbonyl group through a peptide moiety of the glycopeptide, can be immobilized on a substrate that is coated with a polymer compound containing a unit having a primary amino group with high efficiency through the carbonyl group. Particularly, the present inventors have found that a glycopeptide can be immobilized on a substrate with high efficiency and the non-specific adsorption or binding of a substance to be detected can be inhibited effectively when a substrate that is coated with a polymer compound containing a unit having a primary amino group, a unit for retaining hydrophilicity and a unit having a hydrophobic group is used as the substrate for the glycopeptide array, thus completing the present invention.

Specifically, the present invention provides the following inventions.
(1) A glycopeptide array in which a glycopeptide is immobilized on a substrate, wherein the glycopeptide is bound to a molecule having a carbonyl group through a peptide moiety of the glycopeptide, the substrate is coated with a polymer compound containing a unit having a primary amino group, and the glycopeptide is immobilized on the substrate by binding between the carbonyl group and the primary amino group.
(2) The array according to (1), wherein the polymer compound on the substrate further contains a unit having a phosphorylcholine group and a unit having a hydrophobic group.
(3) The array according to (2), wherein the polymer compound is a compound represented by the following general formula [1].

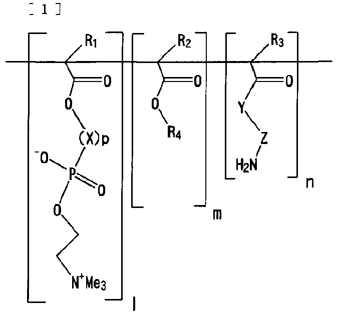

   In the formula, R1, R2 and R3 represent a hydrogen atom or a methyl group and R4 represents a hydrophobic group; X represents an alkyleneoxy group having 1 to 10 carbon atoms, and p represents an integer of 1 to 20; when p is an integer equal to or more than 2 and equal to or less than 20, the repeated X may be the same or may be different; Y is a spacer containing an alkylene glycol residue; Z represents an oxygen atom, NH, NH(C=O)NH or NH(C=S)NH; and 1, m and n are natural numbers (here, the natural numbers refer to integers equal to or more than 1).
(4) The array according to (3), wherein Y in the general formula [1] is represented by the following general formula [2] or [3].

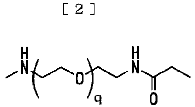

   In the formula, q and r are integers of 1 to 20.

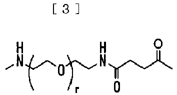

   In the formula, q and r are integers of 1 to 20.
(5) The array according to any one of (1) to (4), wherein the primary amino group in the polymer compound is an oxylamino group and/or a hydrazide group.
(6) The array according to any one of (1) to (5), wherein the content of the unit having a primary amino group in the polymer compound is equal to or more than 20 mol% and equal to or less than 40 mol% with respect to the total unit of the polymer compound.
(7) The array according to (3), wherein X in the general formula [1] is an ethyleneoxy group.
(8) The array according to any one of (1) to (7), wherein a main chain of the polymer compound is a (meth)acrylic skeleton.
(9) The array according to (3), wherein the hydrophobic group R4 is an alkyl group having 2 to 10 carbon atoms.
(10) The array according to (9), wherein the hydrophobic group R4 is a cyclic alkyl group.
(11) The array according to (10), wherein the cyclic alkyl group is a cyclohexyl group.
(12) A method for using the array according to any one of (1) to (11), including: bringing a substance to be detected into contact with a glycopeptide in the array to detect binding between the glycopeptide and the substance to be detected.
(13) The method according to (12), wherein the substance is a material and wherein it is detected that a material to be detected has a capability of binding to a glycopeptide.
(14) The method according to (12), wherein it is detected that there is a substance having a capability of binding to a glycopeptide in a material to be detected brought into contact with the glycopeptide in the array.

### Advantageous Effects of the Invention

When the glycopeptide array of the present invention is used, the interaction between the substance to be detected and the glycopeptide can be detected with high efficiency. Particularly, in the glycopeptide array, by using the substrate that is coated with the polymer compound containing the unit having a primary amino group, the unit for retaining hydrophilicity and the unit having a hydrophobic group, the glycopeptide can be immobilized with high efficiency and non-specific adsorption or binding of the substance to be detected can be inhibited. In addition, since the glycopeptide array of the present invention is immobilized through the carbonyl group which is artificially introduced to the glycopeptide, the carbonyl group can be introduced to an arbitrary position in which there is no influence on the interaction between the substance to be detected and the glycopeptide, and it is advantageous in that the function of the glycopeptide is retained. Such an advantage is a unique effect of the present invention which is difficult to obtain from a method in the related art in which a glycopeptide is immobilized by non-specific adsorption and a method in the related art in which a glycopeptide is immobilized by using a substrate that is subjected to an active ester treatment.

### Brief Description of the Drawings

FIG. 1 is a view showing a MUC1 glycopeptide array used in the present invention.
FIG. 2 is a photo showing results of a detecting reaction of the MUC1 glycopeptide array and six kinds of anti-MUC1 monoclonal antibodies.

### Description of Embodiments

The present invention provides a glycopeptide array in which a glycopeptide is immobilized on a substrate, the glycopeptide is bound to a molecule having a carbonyl group through a peptide moiety of the glycopeptide, the substrate is coated with a polymer compound containing a unit having a primary amino group, and the glycopeptide is immobilized on the substrate by the binding between the carbonyl group and the primary amino group. In addition, a glycopeptide array normally refers to an array in which plural kinds of glycopeptides are immobilized in plural places on a substrate. However, in the specification, the glycopeptide array includes an array in which a single glycopeptide is immobilized only in one position on the substrate, an array in which plural kinds of glycopeptides are immobilized only in one position on the substrate, and an array in which a single glycopeptide is immobilized in plural places on the substrate.

The glycopeptide array of the present invention can be used in the detection of the binding between the substance to be detected and the glycopeptide, and for example, is used in a clinical test, prognosis, drug effect determination, vaccine, early detection, immunological prevention, immunological treatment, safety evaluation for food and agricultural chemicals, home medical care, medical care in a remote place and the like.

Examples of the "carbonyl group" in the molecule having a carbonyl group used in the present invention include a ketone group and an aldehyde group.

The molecule having a carbonyl group may be bound to an N terminal or a C terminal of the peptide moiety of the glycopeptide, and may be bound to other portions. However, preferably, the molecule is bound to the N terminal or the C terminal. In the synthesis of the glycopeptide, when a peptide chain is extended from an amino acid of the C terminal supported by a solid phase resin to the N terminal (in a case of a usual method), it is particularly preferable that the molecule having a carbonyl group be introduced to the N terminal from the viewpoint of synthetic efficiency of the glycopeptide. In this case, the peptide chain with incomplete extension is not immobilized on the substrate since the N terminal is capped during the synthesis, and only the peptide chain extended to a full length is immobilized on the substrate. Therefore, there is an effect of purification of the peptide chain extended to a full length in the aspect in which the molecule having a carbonyl group is introduced to the N terminal.

The substrate of the glycopeptide array of the present invention is coated with the polymer compound containing a unit having a primary amino group. However, the polymer compound preferably further includes a unit for retaining hydrophilicity and a unit having a hydrophobic group. In such a substrate, respectively, the unit for retaining hydrophilicity functions to inhibit physical adsorption (non-specific adsorption) of the substance to be detected to the substrate, and the unit having a hydrophobic group functions to bind the polymer compound to the substrate.

The structure of the unit having a primary amino group contained in the polymer compound used in the present invention is not particularly limited. However, a structure interposing a spacer Y containing a (meth)acrylic residue and an oxylamino residue is preferable as shown in a constituent unit on the right side of constituent units of the following general formula [1]. In the formula, Z represents an oxygen atom when the unit is an oxylamino group, Z represents NH when the unit is a hydrazide group, Z represents NH(C=O)NH when the unit is a semicarbazide group, and Z represents NH(C=S)NH when the unit is a thiosemicarbazide group. While n is originally a natural number, n is represented as a composition ratio of each component in some cases. The structure of the spacer Y containing an alkylene glycol residue is not particularly limited. However, a structure represented by the following general formula [2] or [3] is preferable and the general formula [2] is more preferable. In addition, in the formulae [2] and [3], q and r are integers of 1 to 20.

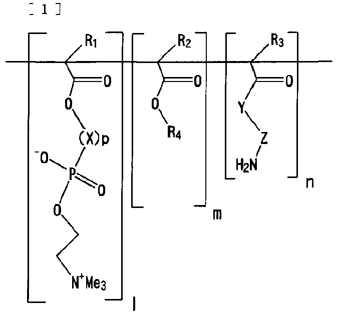

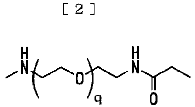

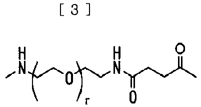

The composition ratio of the unit having a primary amino group contained in the polymer compound used in the present invention (an n ratio with respect to the sum of 1, m and n) is preferably 1 to 94 mol% with respect to the entire unit of the polymer, more preferably 2 to 90 mol%, and most preferably 20 to 40 mol%. When the composition value is less than the lower limit, the glycopeptide cannot be sufficiently immobilized. Moreover, when the composition value is more than the upper limit, non-specific adsorption is increased.

The unit having hydrophilicity contained in the polymer compound is represented as a phosphorylcholine group and the structure thereof is not particularly limited. However, the unit in the general formula [1] most preferably has a structure in which a (meth)acrylic residue and a phosphorylcholine group are bound through an alkylene oxide group X chain having 1 to 10 carbon atoms as shown in a constituent unit on the left side of the constituent units. Among them, X is most preferably an ethyleneoxy group. When the number of the alkylene oxide groups repeated in the formula is an integer of 1 to 20, and the number of repetitions is equal to or more than 2 and equal to or less than 20, the number of carbon atoms in the repeated alkylene oxide group may be the same or may be different. While 1 is originally a natural number, 1 is represented as a composition ratio of each constituent component in some cases.

The composition ratio of the unit having a phosphorylcholine group contained in the polymer compound (an 1 ratio with respect to the sum of 1, m and n) is preferably 5 to 98 mol% with respect to the entire unit of the polymer, more preferably 10 to 80 mol%, and most preferably 10 to 80 mol%. When the composition ratio is less than the lower limit, hydrophilicity becomes weak and non-specific adsorption is increased. On the other hand, when the composition ratio is more than the upper limit, water solubility is increased and thus, it is possibile that the polymer compound in the assay gets eluted.

The structure of the unit having a hydrophobic group contained in the polymer compound used in the present invention is not particularly limited. However, a structure in which a hydrophobic group is bound to a (meth)acrylic group residue is preferable as shown in a constituent unit in the center of the constituent units in the general formula [1]. The hydrophobic group is not particularly limited, and examples thereof include an alkyl group and aromatic series. More preferably, the alkyl group is an alkyl group having 2 to 10 carbon atoms. The structure of the alkyl group is not particularly limited and may be a straight chain, branched or circular structure. Since the unit having a hydrophobic group is contained in the polymer compound, wettability against a hydrophobic substrate, such as plastic, is improved and even coating can be performed. In addition, since the hydrophobicity is increased, the elution of the polymer compound in the assay can be prevented. In the formula, while m is originally a natural number, m is represented as a composition ratio of each constituent in some cases.

The composition ratio of the unit having a hydrophobic group contained in the polymer compound (an m ratio with respect to the sum of 1, m and n) is preferably 10 to 90 mol% with respect to the entire unit of the polymer, more preferably 10 to 80 mol%, and most preferably 20 to 80 mol%. When the composition ratio is more than the upper limit, there is a concern of increasing non-specific adsorption.

A method for synthesizing the polymer compound used in the present invention is not particularly limited. However, from the viewpoint of ease of synthesis, a production method which includes radically copolymerizing at least a monomer in which a primary amino group is protected by a protective group in advance, a monomer having a phosphorylcholine group and a monomer having a hydrophobic group, and removing the protective group from the polymer compound obtained from the radical copolymerizing is preferable. Alternatively, a production method which includes radically copolymerizing at least a monomer having a functional group to which a primary amino group can be introduced, a monomer having a phosphorylcholine group and a monomer having a hydrophobic group, and introducing the primary amino group to the polymer compound obtained from the radical copolymerizing is preferable.

The structure of the monomer in which a primary amino group is protected by a protective group in advance is not particularly limited. However, as shown in the following general formula [4] (in the formula, R3 represents a hydrogen atom or a methyl group; Y represents a spacer containing an alkylene glycol residue; Z represents an oxygen atom or NH; and W represents a protective group), a structure interposing a spacer Y containing an alkylene glycol residue between a (meth)acrylic group and an oxylamino group or a hydrazide group is preferable.

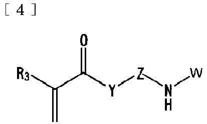

The protective group W is not limited as long as the amino group can be a protective group and an arbitrary one can be used. Among them, a t-butoxycarbonyl group (Boc group), a benzyloxycarbonyl group (Z group, Cbz group), a 9-fluorenylmethoxycarbonyl group (Fmoc group) and the like can be preferably used.

A specific example of the monomer includes one represented by the following formula.

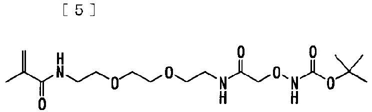

Deprotection can be performed under general conditions when trifluoroacetic acid, hydrochloric acid, and anhydrous hydrogen fluoride are used.

The monomer having a phosphorylcholine group is not particularly limited in structure, and examples thereof include 2-(meth)acryloyloxyethyl phosphorylcholine, 2-(meth)acryloyloxyethoxyethyl phosphorylcholine, 6-(meth)acryloyloxyhexyl phosphorylcholine, 10-(meth)acryloyloxyethoxynonyl phosphorylcholine, and 2-(meth)acryloyloxypropyl phosphorylcholine. However, from the viewpoint of availability, 2-methacryloyloxyethyl phosphorylcholine is preferable.

Specific examples of the monomer having a hydrophobic group include n-butyl(meth)acrylate, iso-butyl(meth)acrylate, sec-butyl(meth)acrylate, t-butyl(meth)acrylate, n-neopentyl(meth)acrylate, iso-neopentyl(meth)acrylate, sec-neopentyl(meth)acrylate, neopentyl(meth)acrylate, cyclohexyl(meth)acrylate, n-hexyl(meth)acrylate, iso-hexyl(meth)acrylate, heptyl(meth)acrylate, n-octyl(meth)acrylate, iso-octyl(meth)acrylate, 2-ethylhexyl(meth)acrylate, n-nonyl(meth)acrylate, iso-nonyl(meth)acrylate, n-decyl(meth)acrylate, iso-decyl(meth)acrylate, n-dodecyl(meth)acrylate, iso-dodecyl(meth)acrylate, n-tridecyl(meth)acrylate, iso-tridecyl(meth)acrylate, n-tetradecyl(meth)acrylate, iso-tetradecyl(meth)acrylate, n-pentadecyl(meth)acrylate, iso-pentadecyl(meth)acrylate, n-hexadecyl(meth)acrylate, iso-hexadecyl(meth)acrylate, n-octadecyl(meth)acrylate, iso-octadecyl(meth)acrylate and isobornyl(meth)acrylate. Among them, cyclohexyl(meth)acrylate, n-butyl methacrylate, n-dodecyl methacrylate and n-octyl methacrylate are preferable.

On the other hand, a method for introducing the primary amino group after the polymer compound is polymerized is not particularly limited. However, a method in which at least the monomer having a phosphorylcholine group, the monomer having a hydrophobic group and the monomer having an alkoxy group are radically copolymerized, and then the alkoxy group introduced to the polymer compound reacts with hydrazine to form a hydrazide group is simple and preferable. As the alkoxy group, a methoxy group, an ethoxy group, a propoxy group, a t-butoxy group, and the like are preferable.

A specific example of the monomer having the alkoxy group includes one represented by the following formula.

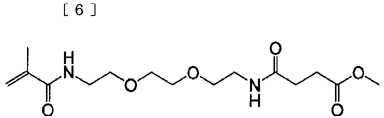

As a synthetic solvent of the polymer compound of the present invention, any solvent can be used as long as each monomer can be dissolved therein. Examples thereof include alcohols such as methanol, ethanol, isopropanol, n-butanol, t-butyl alcohol and n-pentanol, benzene, toluene, tetrahydrofuran, dioxane, dichloromethane, chloroform, cyclohexanone, N,N-dimethylformamide, dimethylsulfoxide, methyl acetate, ethyl acetate, butyl acetate, methyl ethyl ketone, methyl butyl ketone, ethylene glycol monoethyl ether, ethylene glycol monomethyl ether and ethylene glycol monobutyl ether. These solvents are used alone or in combination of two or more thereof.

The polymerization initiator may be any ordinary radical initiator. Examples thereof include azo compounds such as 2,2'-azobisisobutylnitrile (hereinafter referred to as "AIBN") and 1,1'-azobis(cyclohexane-1-carbonitrile), and organic peroxides such as benzoyl peroxide, and lauryl peroxide.

Regarding the molecular weight of the polymer compound used in the present invention, the number average molecular weight is preferably equal to or more than 5,000 and more preferably equal to or more than 10,000 from the viewpoint of ease of separation and purification of the polymer compound and an unreacted monomer.

While deprotection can be performed under general conditions when the above-mentioned trifluoroacetic acid, hydrochloric acid, and anhydrous hydrogen fluoride are used, the time when deprotection is performed is as follows.

The deprotection is generally performed at a stage in which polymerization is completed so that a polymer compound can be produced and the polymer compound can be obtained by performing the deprotection after polymerization is completed.

On the other hand, in the present invention, properties that inhibit the non-specific adsorption of the glycopeptide by coating the surface of the substrate with the polymer compound and properties that immobilize the glycopeptide can be easily imparted.

In this case, the polymer compound having the primary amino group having undergone the deprotection can be applied. However, in forming a polymer material having the primary amino group with a high reactivity into a solution and applying the solution, it can be thought that the primary amino group reacts with the surface and becomes inactivated during the process depending on the circumstances.

Accordingly, preferably, after the polymer compound is purified immediately before the deprotection, and applied onto the surface of the substrate, the deprotection reaction is performed and a state in which the primary amino group is present on the surface of the substrate is made.

The coating of the polymer compound on the surface of the substrate is performed, for example, by dissolving the polymer compound in an organic solvent to prepare a polymer solution having a concentration of 0.05 to 50% by weight, applying the obtained solution on the surface of the substrate by a known method such as dipping or spraying, and then, drying the resultant at room temperature or while heating.

Examples of the organic solvent include alcohols such as ethanol, methanol, isopropanol, n-butanol, t-butyl alcohol, n-pentanol and cyclohexanol, benzene, toluene, tetrahydrofuran, dioxane, dichloromethane, chloroform, acetone, methyl acetate, ethyl acetate, butyl acetate, methyl ethyl ketone, methyl butyl ketone, ethylene glycol monoethyl ether, ethylene glycol monomethyl ether, ethylene glycol monobutyl ether and cyclohexanone. These solvents are used alone or in combination of two or more thereof. Among them, alcohols such as ethanol, methanol, isopropanol, n-butanol, t-butyl alcohol, n-pentanol, cyclohexanol are preferable since a plastic substrate is not deformed and easily dried.

As the substrate used in the present invention, a slide-like substrate, a 96-hole plate, a container and a microfluidic substrate are preferable. Examples thereof include a plastic substrate, a glass substrate and a substrate with a deposited metal film. Specific examples of the plastic substrate include substrates using a polystyrene, cyclic polyolefin polymer, cycloolefin polymer, polypropylene, polyethylene, polysulphone, polyamide, polycarbonate and polymethyl methacrylate, as materials.

Particularly, as a substrate used in fluorescent observation, a cyclic polyolefin polymer and a cycloolefin polymer are used. In the case of using the substrate, when the hydrophobic group of the polymer is a cyclohexyl group, the interaction with the substrate is good, and a good result is obtained in that the adsorption amount is large and the background value is low, compared to the case in which a polymer material having the same composition ratio is applied to another substrate (for example, a polystyrene or glass substrate).

Moreover, when the polymer compound is applied to the cyclic polyolefin polymer, a polymer compound with 100 mol% of an aminooxy monomer cannot be applied. On the other hand, while the polymer compound can be applied to a polystyrene polymer, the non-specific adsorption of a contaminated substance is not inhibited and thus, versatility is poor.

As a method for immobilizing the glycopeptide on the substrate, there are a method for spotting a solution with a glycopeptide dissolved therein by a spotter, a method for aliquoting a solution with a glycopeptide dissolved therein to a container to immobilize the glycopeptide and the like.

As the solution to dissolve the glycopeptide, various buffers are preferably used. There is no particular limitation thereto and examples thereof include sodium carbonate, sodium hydrogen carbonate, potassium phosphate, dibasic potassium phosphate, a tris-hydrochloric acid buffer, tris-acetic acid buffer, PBS buffer, sodium citrate, sodium acetate, HEPES (N-2-hydroxyethylpiperazine-N'-ethanesulphonic acid) buffer, MOPS (3-(N-morpholino)propanesulphonic acid) buffer.

When the glycopeptide is dissolved, the pH of the solution is preferably 2 to 8. The concentration of the glycopeptide in the solution is not particularly limited and a concentration from 0.0001 mg/ml to 10 mg/ml is preferable. The temperature to immobilize the glycopeptide solution is preferably from 0°C to 100°C.

In addition, a method for using the glycopeptide array thus produced according to the present invention also provides a method including bringing a substance to be detected into contact with a glycopeptide in the array to detect binding between the glycopeptide and the substance to be detected. In order to detect the binding, the substance to be detected may be labeled as necessary. The label is not particularly limited as long as the label can be detected, and for example, fluorescent dyes, radioactive materials, chemiluminescent substances, enzymes and coenzymes can be used. Specific examples thereof include fluorescein, rhodamine, dansyl chloride, luciferase, radioisotope, peroxidase, alkaline phosphatase, lysozyme, biotin/avidin and the like.

Furthermore, the present invention is a method for using the glycopeptide array thus produced and provides a method for detecting that a material to be detected has a capability of binding to a glycopeptide by bringing the material to be detected into contact with the glycopeptide in the array and detecting the binding between the glycopeptide and the material to be detected, and a method for detecting that there is a substance having a capability of binding to a glycopeptide in a material to be detected by bringing the material to be detected into contact with the glycopeptide in the array and detecting binding between the glycopeptide and the material to be detected. In order to detect the binding, the material to be detected may be labeled as necessary. The label is not particularly limited as long as the label can be detected, and for example, fluorescent dyes, radioactive materials, chemiluminescent substances, enzymes and coenzymes can be used. Specific examples thereof include fluorescein, rhodamine, dansyl chloride, luciferase, radioisotope, peroxidase, alkaline phosphatase, lysozyme, biotin/avidin and the like.

### Examples

Hereinafter, the present invention will be described in detail based on Examples and Comparative Examples. However, the present invention is not limited to the following examples.

### [Example 1] Synthesis of MUC1 glycopeptide

An oligopeptide having the following amino acid was synthesized according to previous literature (Japanese Unexamined Patent Application, First Publication No. 2006-63055).

Sequence 1: 5-oxo linker-GVTSAPDTRPAPGSTAPPAHGVT-NH₂/Sequence Number: 1
Sequence 2: 5-oxo linker-STAPPAHGVTGVTSAPDTRPAPGSTA-NH₂/Sequence Number: 2

Monosaccharide N-acetyl galactosamine (Tn), disaccharide galactose-N-acetylgalactosamine (T:Galβ1 → 3GalNAc) and trisaccharide N-acetylneuramic acid-galactose-N-acetylgalactosamine (Sialyl-T: NeuAcα2 → 3Galβ1 → 3GalNAc) were bound to a hydroxyl group of threonine (T) and serine (S) in 8th, 14th and 15th positions from the side of 5-oxyhexanoyl introduced to the N(amino) terminal in Sequence 1 to produce a compound described in the following Table 1.

In the same manner, monosaccharide N-acetyl galactosamine (Tn), disaccharide galactose-N-acetylgalactosamine (T:Galβ1 → 3GalNAc) and trisaccharide N-acetylneuramic acid-galactose-N-acetylgalactosamine (Sialyl-T: NeuAcα2 → 3Galβ1 → 3GalNAc) were bound to a hydroxyl group of threonine (T) and serine (S) in 13th, 14th and 18th positions from the side of 5-oxyhexanoyl introduced to the N(amino) terminal in Sequence 2 to produce a compound described in the following Table 1.

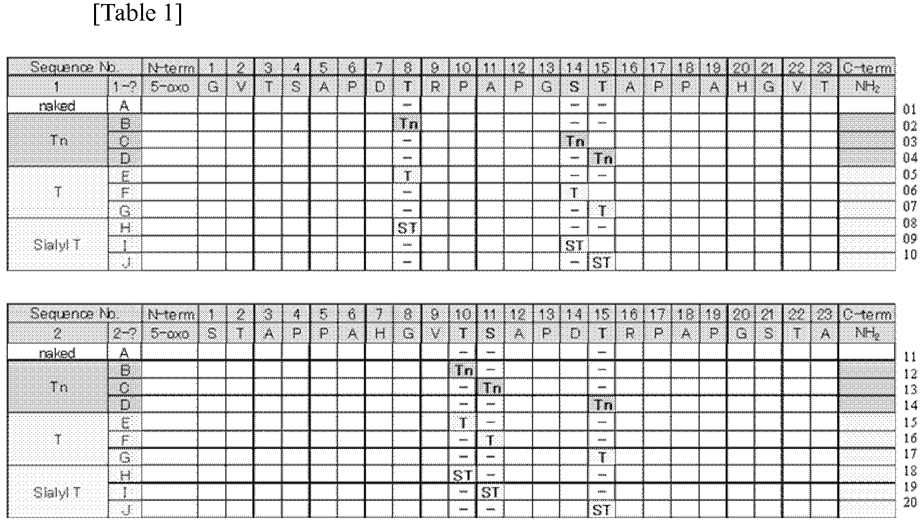

### [Example 2] Synthesis of Polymer

### (1) Synthesis of Polymer Compound

Each of 2-methacryloyloxyethyl phosphorylcholine (MPC), n-butyl methacrylate (BMA) and N-[2-[2-[2-(t-butoxycarbonyl aminooxy acetylamino)ethoxy]ethoxy] ethyl]-methacrylamide (OA, compound represented in formula [5]) was sequentially dissolved in ethanol so that the concentration thereof was 0.25 mol/L, 0.55 mol/L and 0.20 mol/L to produce a mixed monomer solution. AIBN was added therein so that the concentration is 0.01 mol/L and stirred until being even. Then, the mixture was allowed to react at 60°C for 6 hours under an argon gas atmosphere, and then, the reaction solution was dropped in diethyl ether to collect a precipitate. The obtained polymer compound was measured using 1H-NMR to calculate the composition ratio of the polymer compound. The results are shown in Table 2.

**[Table 2]**

| MPC | BMA | OA |
|---|---|---|
| 26 | 66 | 8 |
| (mol%) | | |

### (2) Deprotection of Polymer Compound

The polymer compound was treated with a 2N HCl-dioxane-ethanol solution for 4 hours at room temperature to remove a Boc group. After the deprotection, the 1H-NMR of the polymer compound was measured to confirm the deprotection from the fact that a peak caused by trimethyl of the Boc group disappeared.

### (3) Production of Substrate for Immobilizing Glycopeptide

A saturated cyclic polyolefin resin (hydrogenated product of ring-opening polymer of 5-methyl-2-norbornene, MFR (melt flow rate): 21 g/10 minutes, rate of hydrogenation: substantially 100%, thermal deformation temperature: 123°C) was molded in a glass-slide-like shape (size: 75 mm x 25 mm x 1 mm) to prepare a solid phase substrate. The solid phase substrate was dipped in the 0.3 % by weight ethanol of the copolymer of 2-methacryloyloxyethyl phosphorylcholine (MPC), n-butyl methacrylate (BMA) and N-[2-[2-[2-(t-butoxycarbonyl aminooxy acetylamino)ethoxy]ethoxy]ethyl]-methacrylamide (OA) (each group is 26:66:8 in terms of mol%) and dried so that the polymer substance was introduced onto the surface of the substrate.

### [Example 3] Production of MUC1 Glycopeptide Array

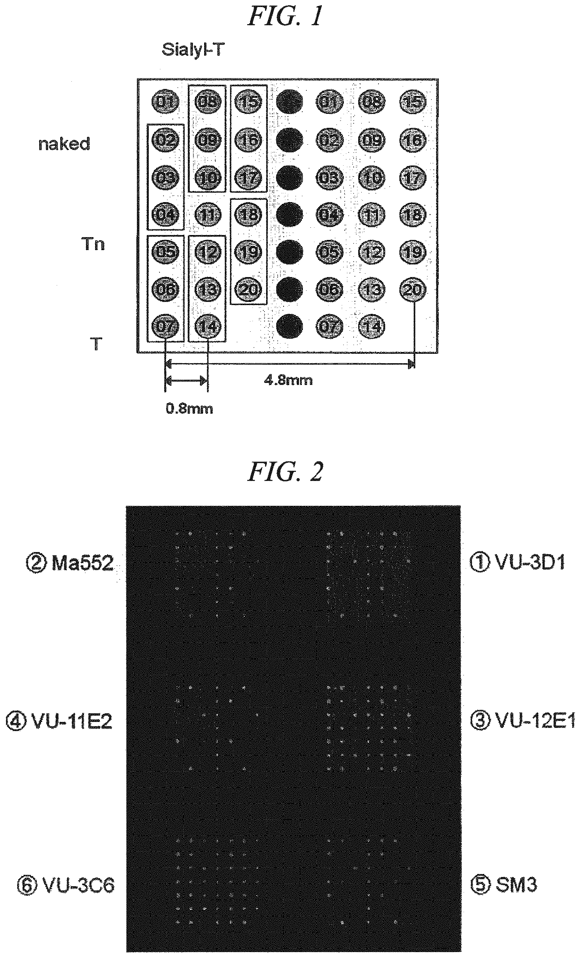

A solution which was prepared to have a concentration of 0.5 mM in a 0.5M acetic acid buffer was spotted using an automatic spotter as shown in FIG. 1 and kept at 80°C for 1 hour so as to immobilize the obtained glycopeptide. After the immobilization, the substrate was washed with ultrapure water.

### [Example 4] Detection of MUC1 Glycopeptide Array

The MUC1 glycopeptide array was measured by the following method. Solutions of six kinds of anti-MUC1 monoclonal antibodies (VU-3D1, Ma552, VU-12E1, VU11E2, SM2 and VU-3C6) were aliquoted in 100 µL to each spot portion and allowed to react at room temperate for 1 hour. After the reaction, the substrate was washed with the following washing solution 5 times.

Washing solution: 50 mM Tris·HCl (pH 7.5), 100 mM NaCl, 1 mM CaCl₂, MnCl₂, MgCl₂, 0.05% Triton X-100 (all reagents manufactured by Wako Pure Chemical Industries, Ltd.)

Next, 1 µg/mL of Cy3-labeled goat anti-mouse IgG (H+L) was aliquoted in 100 µL to each spot and allowed to react at room temperate for 1 hour. After the reaction, the substrate was washed with the following washing solution 5 times.

Fluorescence was measured using a microarray scanner "ScanArray" (manufactured by Packard Biochip Technologies).

As a result, it was confirmed that the reactivity with the glycopeptide used as an antigen was different in the six kinds of antibodies (FIG. 2). When the VU-3C6 antibody or VU-12E1 antibody was used, fluorescent emission in all spots was observed. Contrarily, when the Ma552 antibody, VU-11E2 antibody, VU-3D1 antibody or SM3 antibody was used, the reactivity was decreased along with the extension of a modified sugar chain.

Hereinafter, reference examples of the polymer compound that coats the substrate will be shown.

### <Reference Example 1> Comparison of Aminooxy Group Content (96-well plate)

### (1) Production of Sugar Chain-Capturing Substrate

A 96-well plate was used as a substrate having a sugar chain-capturing polymer material formed on the surface. The 96-well plate formed of a cyclic polyolefin resin and manufactured by Sumitomo Bakelite Co., Ltd. was used.

Using a 2-methacryloyloxyethyl phosphorylcholine-butylmethacrylate-N-[2-[2-[2-(t-butoxycarbonyl aminooxy acetylamino)ethoxy]ethoxy]ethyl]-methacrylamide copolymer as the sugar chain-capturing polymer compound, 150 µL of 1.0 % by weight of an ethanol solution with the polymer compound was aliquoted to each well of the 96-well plate, and kept for 30 minutes, then, the solution was extracted and dried, and a solvent was evaporated to coat the surface of the substrate. As the polymer compound, three kinds of compounds each having the content of an aminooxy group of 14, 16 and 20% were synthesized and used. After the drying, 2M HCl was aliquoted, the plate was treated at 37°C for 2 hours, and a Boc group was deprotected so as to produce a 96-well plate having a sugar chain-capturing polymer compound on the surface.

### (2) Sugar Chain Immobilization

Disaccharide lactose (Wako Pure Chemical Industries, Ltd., 124-00092) was prepared to have a concentration of 1 mg/mL using a 300 mM sodium acetate buffer (pH 4.0) so as to produce a lactose solution. The produced lactose solution was aliquoted in 200 µL to the 96-well plate for capturing a sugar chain and allowed to react at 65°C for 16 hours. After the reaction, unreacted lactose was removed by a solvent.

### (3) Detection of Immobilized Sugar Chain

A biotin-labeled RCA120 lectin which specifically reacts with lactose (Biotin-labeled RCA120 lectin (manufactured by Vector Laboratories, Inc., B-1085)) was prepared to have a concentration of 0.5 µg/mL using the following solvent.

Solvent: 50 mM Tris·HCl (pH 7.5), 100 mM NaCl, 1 mM CaCl₂, MnCl₂, MgCl₂, 0.05% Tween 20 (all regents manufactured by Wako Pure Chemical Industries, Ltd.)

The solution was aliquoted in 100 µL to each well and allowed to react at room temperature for 2 hours. After the reaction, the inside of each well was washed with the following washing solution 3 times.

Washing solution: 50 mM Tris·HCl (pH 7.5), 100 mM NaCl, 1 mM CaCl₂, MnCl₂, MgCl₂, 0.05% Triton X-100 (all reagents manufactured by Wako Pure Chemical Industries, Ltd.)

A peroxidase-labeled avidin (manufactured by MP Biomedicals LLC., 191370) was prepared to have a concentration of 0.5 µg/mL using the following solvent.

Solvent: 50 mM Tris·HCl (pH 7.5), 100 mM NaCl, 1 mM CaCl₂, MnCl₂, MgCl₂, 0.05% Tween 20 (all regents manufactured by Wako Pure Chemical Industries, Ltd.)

A peroxidase solution was aliquoted in 100 µL to the 96-well plate on which the lactose was immobilized and allowed to react at room temperature for 1 hour. After the reaction ended, each well was washed with the washing solution 3 times.

In the measurement of the amount of the solid-phased peroxidase, the peroxidase was allowed to develop color for 15 minutes using a chromophoric kit for peroxidase (manufactured by Sumitomo Bakelite Co., Ltd., ML-1120T), and then, subjected to measurement of absorbance at a measurement wavelength of 450 nm using a microplate reader (Infinit 200 manufactured by Tecan Group Ltd.).

The results are shown in Table 3.

**[Table 3]**

| Aminooxy Content (%) | Absorbance |
|---|---|
| 14 | 0.981 |
| 16 | 1.362 |
| 20 | 1.803 |

### <Reference Example 2> Comparison of Hydrophobicity Residue Structure (Background Comparison, 96-Well Plate)

The 96-well microplate (manufactured by Sumitomo Bakelite Co., Ltd.) formed of cyclic olefin used in Reference Example 1 was used. A polymer compound synthesized by changing butylmethacrylate of the sugar chain-capturing polymer compound in Reference Example 1 to cyclohexyl methacrylate or n-hexyl methacrylate was applied in the same manner as in Reference Example 1 to deprotect a Boc group. A biotin-labeled RCA120 lectin was allowed to react, and further, a peroxidase-labeled avidin was allowed to react. Then, the resultant was allowed to develop color using a peroxidase chromophoric kit and subjected to measurement of absorbance.

The results are shown in Table 4.

**[Table 4]**

| Hydrophobic group | Background absorbance |
|---|---|
| Butyl | 0.211 |
| Cyclohexyl | 0.125 |
| n-hexyl | 0.250 |

Low background values were obtained in the butyl group and n-hexyl group, which are straight chain alkyl groups. However, a lower background value was obtained in the cyclohexyl group, which is a cyclic alkyl group.

### <Reference Example 3> Comparison of Hydrophobicity Residue Structure (signal value comparison, 96-well plate)

The 96-well microplate (manufactured by Sumitomo Bakelite Co., Ltd.) formed of cyclic olefin used in Reference Examples 1 and 2 was used. A polymer compound synthesized by changing butylmethacrylate of the sugar chain-capturing polymer compound in Reference Example 1 to cyclohexyl methacrylate was applied in the same manner as in Reference Example 1 to deprotect a Boc group.

### (1) Sugar Chain Immobilization

Mannotriose (Dextra, M336) was prepared using the following solvent to have a concentration of 10 µg/mL. The solution was aliquoted in 100 µL to each well of the 96-well plate and allowed to react at 80°C for 1 hour.

Solvent: 2% AcOH/CH₃CN:water=75:25

After the reaction ended, the plate was washed and unreacted mannotriose was removed.

### (2) Detection of Immobilized Sugar Chain

A biotin-labeled ConA lectin which specifically reacts with mannotriose (manufactured by Sigma-Aldrich Co. LLC., C2272) was prepared to have a concentration of 250 ng/mL using the following solvent.

Solvent: 50 mM Tris·HCl (pH 7.5), 100 mM NaCl, 1 mM CaCl₂, MnCl₂, MgCl₂, 0.05% Tween 20

The solution was aliquoted in 100 µL to each well and allowed to react at room temperature for 2 hours. After the reaction, the inside of each well was washed with the following washing solution 3 times.

Washing solution: 50 mM Tris·HCl (pH 7.5), 100 mM Nail, 1 mM CaCl2, MnCl₂, MgCl2, 0.05% Triton X-100

A peroxidase-labeled avidin (manufactured by MP Biomedicals LLC., 191370) was prepared to have a concentration of 0.5 µg/mL using the following solvent.

Solvent: 50 mM Tris·HCl (pH 7.5), 100 mM NaCl, 1 mM CaCl₂, MnCl₂, MgCl₂, 0.05% Tween 20

A peroxidase solution was aliquoted in 100 µL to the 96-well plate on which the lactose was immobilized and allowed to react at room temperature for 1 hour. After the reaction ended, each well was washed with the washing solution 3 times. In the measurement of the amount of the solid-phased peroxidase, the peroxidase was allowed to develop color for 15 minutes using TMB (manufactured by Bio-Rad Laboratories, Inc., 172-1067), and then, subjected to measurement of absorbance at 450 nm.

The results are shown in Table 5.

**[Table 5]**

| Hydrophobic group | Absorbance |
|---|---|
| Butyl | 1.333 |
| Cyclohexyl | 3.028 |

A high absorbance value could be obtained in the cyclohexyl group, which is a cyclic alkyl group.

### Industrial Applicability

When the glycopeptide array of the present invention is used, an interaction between a substance to be detected and a glycopeptide can be detected with high efficiency. Since glycopeptides are significantly related to many vital phenomena and clinical conditions such as cancer and infections, the glycopeptide array of the present invention contributes significantly to, for example, the development of a new diagnostic method and medical treatment.

### Sequence Listing Free Text

Seq. Nos. 1 to 2
<223> Artificially synthesized peptide sequence

## Claims

1. A glycopeptide array in which a glycopeptide is immobilized on a substrate, wherein the glycopeptide is bound to a molecule having a carbonyl group through a peptide moiety of the glycopeptide, the substrate is coated with a polymer compound containing a unit having a primary amino group, and the glycopeptide is immobilized on the substrate by binding between the carbonyl group and the primary amino group.

2. The array according to Claim 1,
wherein the polymer compound on the substrate further contains a unit having a phosphorylcholine group and a unit having a hydrophobic group.

3. The array according to Claim 2,
wherein the polymer compound is a compound represented by the following general formula [1]: 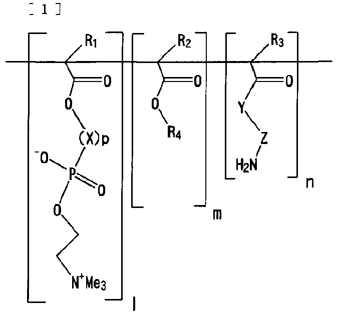 (in the formula, R1, R2 and R3 represent a hydrogen atom or a methyl group and R4 represents a hydrophobic group; X represents an alkyleneoxy group having 1 to 10 carbon atoms, and p represents an integer of 1 to 20; when p is an integer equal to or more than 2 and equal to or less than 20, the repeated X may be the same or may be different; Y is a spacer containing an alkylene glycol residue; Z represents an oxygen atom, NH, NH(C=O)NH or NH(C=S)NH; and 1, m and n are natural numbers).

4. The array according to Claim 3,
wherein Y in the general formula [1] is represented by the following general formula [2] or [3]: 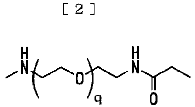 (in the formula, q and r are integers of 1 to 20) 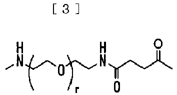 (in the formula, q and r are integers of 1 to 20).

5. The array according to any one of Claims 2 to 4,
wherein the primary amino group in the polymer compound is an oxylamino group and/or a hydrazide group.

6. The array according to Claim 5,
wherein the content of the unit having a primary amino group in the polymer compound is equal to or more than 20 mol% and equal to or less than 40 mol% with respect to the total unit of the polymer compound.

7. The array according to Claim 3,
wherein X in the general formula [1] is an ethyleneoxy group.

8. The array according to any one of Claims 1 to 7,
wherein a main chain of the polymer compound is a (meth)acrylic skeleton.

9. The array according to Claim 3,
wherein the hydrophobic group R4 is an alkyl group having 2 to 10 carbon atoms.

10. The array according to Claim 9,
wherein the hydrophobic group R4 is a cyclic alkyl group.

11. The array according to Claim 10,
wherein the cyclic alkyl group is a cyclohexyl group.

12. A method for using the array according to any one of Claims 1 to 11 comprising:
bringing a substance to be detected into contact with a glycopeptide in the array to detect binding between the glycopeptide and the substance to be detected.

13. The method according to Claim 12,
wherein the substance is a material and wherein it is detected that a material to be detected has a capability of binding to a glycopeptide.

14. The method according to Claim 12,
wherein it is detected that there is a substance having a capability of binding to a glycopeptide in a material to be detected brought into contact with the glycopeptide in the array.

## Patentansprüche

1. Glykopeptid-Anordnung, in der ein Glykopeptid auf einem Substrat immobilisiert ist,
wobei das Glykopeptid an ein Molekül mit einer Carbonylgruppe über einen Peptidrest des Glykopeptids gebunden ist, das Substrat mit einer Polymerverbindung, die eine Einheit mit einer primären Aminogruppe enthält, beschichtet ist und das Glykopeptid auf dem Substrat durch Bindung zwischen der Carbonylgruppe und der primären Aminogruppe immobilisiert ist.

2. Anordnung gemäß Anspruch 1,
wobei die Polymerverbindung auf dem Substrat ferner eine Einheit mit einer Phosphorylcholin-Gruppe und eine Einheit mit einer hydrophoben Gruppe enthält.

3. Anordnung gemäß Anspruch 2,
wobei die Polymerverbindung eine Verbindung ist, die durch die nachstehende allgemeine Formel [1] repräsentiert wird: 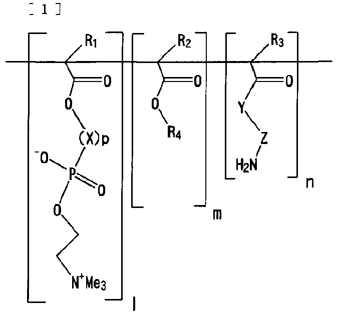 (in der Formel repräsentieren R1, R2 und R3 ein Wasserstoffatom oder eine Methylgruppe und R4 repräsentiert eine hydrophobe Gruppe; X repräsentiert eine Alkylenoxygruppe mit 1 bis 10 Kohlenstoffatomen, und p repräsentiert eine ganze Zahl von 1 bis 20; wenn p eine ganze Zahl ist, die gleich zu oder größer als 2 und gleich zu oder kleiner als 20 ist, können die wiederholten X gleich oder verschieden sein; Y ist ein Abstandhalter, der einen Alkylenglykolrest enthält; Z repräsentiert ein Sauerstoffatom, NH, NH(C=O)NH oder NH(C=S)NH; und 1, m und n sind natürliche Zahlen).

4. Anordnung gemäß Anspruch 3,
wobei Y in der allgemeinen Formel [1] durch die nachstehende allgemeine Formel [2] oder [3] repräsentiert wird: 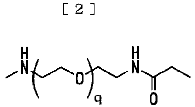 (in der Formel sind q und r ganze Zahlen von 1 bis 20) 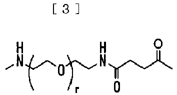 (in der Formel sind q und r ganze Zahlen von 1 bis 20).

5. Anordnung gemäß einem jeglichen der Ansprüche 2 bis 4,
wobei die primäre Aminogruppe in der Polymerverbindung eine Oxylaminogruppe und/oder eine Hydrazidgruppe ist.

6. Anordnung gemäß Anspruch 5,
wobei der Gehalt der Einheit mit einer primären Aminogruppe in der Polymerverbindung gleich zu oder größer als 20 mol% und gleich zu oder kleiner als 40 mol% hinsichtlich der gesamten Einheiten der Polymerverbindung ist.

7. Anordnung gemäß Anspruch 3,
wobei X in der allgemeinen Formel [1] eine Ethylenoxygruppe ist.

8. Anordnung gemäß einem jeglichen der Ansprüche 1 bis 7,
wobei eine Hauptkette der Polymerverbindung ein (Meth)acryl-Skelett ist.

9. Anordnung gemäß Anspruch 3,
wobei die hydrophobe Gruppe R4 eine Alkylgruppe mit 2 bis 10 Kohlenstoffatomen ist.

10. Anordnung gemäß Anspruch 9,
wobei die hydrophobe Gruppe R4 eine cyclische Alkylgruppe ist.

11. Anordnung gemäß Anspruch 10,
wobei die cyclische Alkylgruppe eine Cyclohexylgruppe ist.

12. Verfahren zum Verwenden der Anordnung gemäß einem jeglichen der Ansprüche 1 bis 11, umfassend:
In-Kontakt-Bringen einer nachzuweisenden Substanz mit einem Glykopeptid in der Anordnung, um ein Binden zwischen dem Glykopeptid und der nachzuweisenden Substanz nachzuweisen.

13. Verfahren gemäß Anspruch 12,
wobei die Substanz ein Material ist und wobei nachgewiesen wird, dass ein nachzuweisendes Material die Fähigkeit eines Bindens an ein Glykopeptid aufweist.

14. Verfahren gemäß Anspruch 12,
wobei nachgewiesen wird, dass es eine Substanz, die die Fähigkeit eines Bindens an ein Glykopeptid aufweist, in einem nachzuweisenden Material gibt, das mit dem Glykopeptid in der Anordnung in Kontakt gebracht wurde.

## Revendications

1. Réseau de glycopeptides dans lequel un glycopeptide est immobilisé sur un substrat,
tandis que le glycopeptide est lié à une molécule ayant un groupe carbonyle par l'intermédiaire d'une portion peptidique du glycopeptide, le substrat est revêtu avec un composé polymère contenant une unité ayant un groupe amino primaire, et le glycopeptide est immobilisé sur le substrat par liaison entre le groupe carbonyle et le groupe amino primaire.

2. Réseau selon la revendication 1,
dans lequel le composé polymère sur le substrat contient en outre une unité ayant un groupe phosphorylcholine et une unité ayant un groupe hydrophobe.

3. Réseau selon la revendication 2,
dans lequel le composé polymère est un composé représenté par la formule générale [1] suivante: 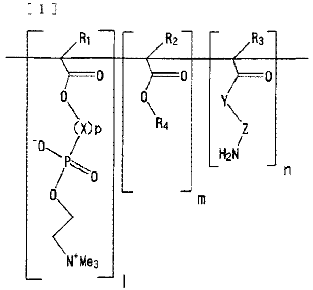 (dans la formule, R1, R2 et R3 représentent un atome d'hydrogène ou un groupe méthyle et R4 représente un groupe hydrophobe; X représente un groupe alkylèneoxy ayant 1 à 10 atomes de carbone, et p représente un entier de 1 à 20; lorsque p est un entier égal ou supérieur à 2 et égal ou inférieur à 20, les X répétés peuvent être les mêmes ou être différents; Y est un espaceur contenant un résidu d'alkylène glycol; Z représente un atome d'oxygène, NH, NH(C=O)NH ou NH(C=S)NH; et 1, m et n sont des nombres naturels).

4. Réseau selon la revendication 3,
dans lequel Y dans la formule générale [1] est représenté par la formule générale [2] ou [3]: 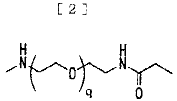 (dans la formule, q et r sont des entiers de 1 à 20) 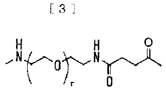 (dans la formule q et r sont des entiers de 1 à 20).

5. Réseau selon l'une quelconque des revendications 2 à 4,
dans lequel le groupe amino primaire dans le composé polymère est un groupe oxylamino et/ou un groupe hydrazide.

6. Réseau selon la revendication 5,
dans lequel la teneur de l'unité ayant un groupe amino primaire dans le composé polymère est égale ou supérieure à 20 % en moles et égale ou inférieure à 40 % en moles par rapport à l'unité totale du composé polymère.

7. Réseau selon la revendication 3,
dans lequel X dans la formule générale [1] est un groupe éthylèneoxy.

8. Réseau selon l'une quelconque des revendications 1 à 7,
dans lequel une chaîne principale du composé polymère est un squelette (méth)acrylique.

9. Réseau selon la revendication 3,
dans lequel le groupe hydrophobe R4 est un groupe alkyle ayant 2 à 10 atomes de carbone.

10. Réseau selon la revendication 9,
dans lequel le groupe hydrophobe R4 est un groupe alkyle cyclique.

11. Réseau selon la revendication 10,
dans lequel le groupe alkyle cyclique est un groupe cyclohexyle.

12. Procédé pour l'utilisation du réseau selon l'une quelconque des revendications 1 à 11, comprenant:
la mise en contact d'une substance à détecter avec un glycopeptide dans le réseau pour détecter la liaison entre le glycopeptide et la substance à détecter.

13. Procédé selon la revendication 12,
dans lequel la substance est une matière et dans lequel on détecte qu'une matière à détecter a une aptitude à se lier à un glycopeptide.

14. Procédé selon la revendication 12,
dans lequel on détecte qu'il existe une substance ayant une aptitude à se lier à un glycopeptide dans une matière à détecter mise en contact avec le glycopeptide dans le réseau.


### SEQUENCE LISTING

<110> SUMITOMO BAKELITE CO., LTD.
<120> GLYCOPEPTIDE ARRAY
<130> 837-5
<140> EP 11843001.6
   <141> 2011-11-22
<150> JP 20100264099
   <151> 2010-11-26
<160> 2
<170> PatentIn version 3.5
<210> 1
   <211> 23
   <212> PRT
   <213> Artificial Sequence
<220>
   <223> Artificially synthesized peptide sequence
<400> 1
   Gly Val Thr Ser Ala Pro Asp Thr Arg Pro Ala Pro Gly Ser Thr Ala
   1               5                   10                  15
   Pro Pro Ala His Gly Val Thr
               20
<210> 2
   <211> 26
   <212> PRT
   <213> Artificial Sequence
<220>
   <223> Artificially synthesized peptide sequence
<400> 2
   Ser Thr Ala Pro Pro Ala His Gly Val Thr Gly Val Thr Ser Ala Pro
   1               5                   10                  15
   Asp Thr Arg Pro Ala Pro Gly Ser Thr Ala
               20                  25